# EUROPÄISCHE PATENTANMELDUNG

(11) **EP 1 829 894 A1**
(43) Veröffentlichungstag der Anmeldung: **05.09.2007**
(21) Anmeldenummer: 06002944.4
(22) Anmeldetag: 14.02.2006
(51) Int. Cl.: C07K 16/18

(54) **Monoklonale Antikörper gegen Kollagen XVII**

(71) Anmelder: Universitätsklinikum Freiburg, 79106 Freiburg (DE)
(72) Erfinder: Bruckner-Tuderman, Leena, Prof.Dr., 79104 Freiburg (DE); Hofmann, Silke, Dr., 79117 Freiburg (DE); Sorokin, Lydia, Prof .Dr., 58097 Hagen (DE)
(74) Vertreter: Kalhammer, Georg

(57) **Zusammenfassung**

Die vorliegende Erfindung betrifft monoklonale Antikörper gegen menschliches Kollagen XVII, die dadurch gekennzeichnet sind, dass sie die Transmembranform von Kollagen XVII erkennen.

SEQUENCE LISTING

<110>  Universitaetsklinikum Freiburg

<120>  Monoklonale Antikörper gegen Kollagen XVII

<130>  Kollagen XVII

<160>  3

<170>  PatentIn version 3.2

<210>  1
<211>  1497
<212>  PRT
<213>  homo sapiens

<400>  1

Met Asp Val Thr Lys Lys Asn Lys Arg Asp Gly Thr Glu Val Thr Glu
1               5                   10                  15

Arg Ile Val Thr Glu Thr Val Thr Thr Arg Leu Thr Ser Leu Pro Pro
            20                  25                  30

Lys Gly Gly Thr Ser Asn Gly Tyr Ala Lys Thr Ala Ser Leu Gly Gly
        35                  40                  45

Gly Ser Arg Leu Glu Lys Gln Ser Leu Thr His Gly Ser Ser Gly Tyr
    50                  55                  60

Ile Asn Ser Thr Gly Ser Thr Arg Gly His Ala Ser Thr Ser Ser Tyr
65                  70                  75                  80

Arg Arg Ala His Ser Pro Ala Ser Thr Leu Pro Asn Ser Pro Gly Ser
                85                  90                  95

Thr Phe Glu Arg Lys Thr His Val Thr Arg His Ala Tyr Glu Gly Ser
            100                 105                 110

Ser Ser Gly Asn Ser Ser Pro Glu Tyr Pro Arg Lys Glu Phe Ala Ser
        115                 120                 125

Ser Ser Thr Arg Gly Arg Ser Gln Thr Arg Glu Ser Glu Ile Arg Val
    130                 135                 140

Arg Leu Gln Ser Ala Ser Pro Ser Thr Arg Trp Thr Glu Leu Asp Asp
145                 150                 155                 160

Val Lys Arg Leu Leu Lys Gly Ser Arg Ser Ala Ser Val Ser Pro Thr
                165                 170                 175

Arg Asn Ser Ser Asn Thr Leu Pro Ile Pro Lys Lys Gly Thr Val Glu
            180                 185                 190

Thr Lys Ile Val Thr Ala Ser Ser Gln Ser Val Ser Gly Thr Tyr Asp
        195                 200                 205

Ala Thr Ile Leu Asp Ala Asn Leu Pro Ser His Val Trp Ser Ser Thr
    210                 215                 220

Leu Pro Ala Gly Ser Ser Ile Gly Thr Tyr His Asn Asn Met Thr Thr
225                 230                 235                 240

Gln Ser Ser Ser Leu Leu Asn Thr Asn Ala Tyr Ser Ala Gly Ser Val
                245                 250                 255

Phe Gly Val Pro Asn Asn Met Ala Ser Cys Ser Pro Thr Leu His Pro
            260                 265                 270

Gly Leu Ser Thr Ser Ser Ser Val Phe Gly Met Gln Asn Asn Leu Ala
        275                 280                 285

Pro Ser Leu Thr Thr Leu Ser His Gly Thr Thr Thr Thr Ser Thr Ala
    290                 295                 300

Tyr Gly Val Lys Lys Asn Met Pro Gln Ser Pro Ala Ala Val Asn Thr
305                 310                 315                 320

Gly Val Ser Thr Ser Ala Ala Cys Thr Thr Ser Val Gln Ser Asp Asp
                325                 330                 335

Leu Leu His Lys Asp Cys Lys Phe Leu Ile Leu Glu Lys Asp Asn Thr
            340                 345                 350

Pro Ala Lys Lys Glu Met Glu Leu Leu Ile Met Thr Lys Asp Ser Gly
        355                 360                 365

Lys Val Phe Thr Ala Ser Pro Ala Ser Ile Ala Ala Thr Ser Phe Ser
    370                 375                 380

Glu Asp Thr Leu Lys Lys Glu Lys Gln Ala Ala Tyr Asn Ala Asp Ser
385                 390                 395                 400

Gly Leu Lys Ala Glu Ala Asn Gly Asp Leu Lys Thr Val Ser Thr Lys
                405                 410                 415

Gly Lys Thr Thr Thr Ala Asp Ile His Ser Tyr Ser Ser Ser Gly Gly
            420                 425                 430

Gly Gly Ser Gly Gly Gly Gly Gly Val Gly Gly Ala Gly Gly Gly Pro
        435                 440                 445

Trp Gly Pro Ala Pro Ala Trp Cys Pro Cys Gly Ser Cys Cys Ser Trp
    450                 455                 460

Trp Lys Trp Leu Leu Gly Leu Leu Leu Thr Trp Leu Leu Leu Leu Gly
465                 470                 475                 480

Leu Leu Phe Gly Leu Ile Ala Leu Ala Glu Glu Val Arg Lys Leu Lys
                485                 490                 495

Ala Arg Val Asp Glu Leu Glu Arg Ile Arg Arg Ser Ile Leu Pro Tyr
            500                 505                 510

Gly Asp Ser Met Asp Arg Ile Glu Lys Asp Arg Leu Gln Gly Met Ala
        515                 520                 525

Pro Ala Ala Gly Ala Asp Leu Asp Lys Ile Gly Leu His Ser Asp Ser
    530                 535                 540

Gln Glu Glu Leu Trp Met Phe Val Arg Lys Lys Leu Met Met Glu Gln
545                 550                 555                 560

Glu Asn Gly Asn Leu Arg Gly Ser Pro Gly Pro Lys Gly Asp Met Gly
                565                 570                 575

Ser Pro Gly Pro Lys Gly Asp Arg Gly Phe Pro Gly Thr Pro Gly Ile
            580                 585                 590

Pro Gly Pro Leu Gly His Pro Gly Pro Gln Gly Pro Lys Gly Gln Lys
        595                 600                 605

Gly Ser Val Gly Asp Pro Gly Met Glu Gly Pro Met Gly Gln Arg Gly
    610                 615                 620

Arg Glu Gly Pro Met Gly Pro Arg Gly Glu Ala Gly Pro Pro Gly Ser
625                 630                 635                 640

Gly Glu Lys Gly Glu Arg Gly Ala Ala Gly Glu Pro Gly Pro His Gly
                645                 650                 655

Pro Pro Gly Val Pro Gly Ser Val Gly Pro Lys Gly Ser Ser Gly Ser
            660                 665                 670

Pro Gly Pro Gln Gly Pro Pro Gly Pro Val Gly Leu Gln Gly Leu Arg
        675                 680                 685

Gly Glu Val Gly Leu Pro Gly Val Lys Gly Asp Lys Gly Pro Val Gly
    690                 695                 700

Pro Pro Gly Pro Lys Gly Asp Gln Gly Glu Lys Gly Pro Arg Gly Leu
705                 710                 715                 720

Thr Gly Glu Pro Gly Met Arg Gly Leu Pro Gly Ala Val Gly Glu Pro
                725                 730                 735

Gly Ala Lys Gly Ala Met Gly Pro Ala Gly Pro Asp Gly His Gln Gly
            740                 745                 750

Pro Arg Gly Glu Gln Gly Leu Thr Gly Met Pro Gly Ile Arg Gly Pro
        755                 760                 765

Pro Gly Pro Ser Gly Asp Pro Gly Lys Pro Gly Leu Thr Gly Pro Gln
    770                 775                 780

Gly Pro Gln Gly Leu Pro Gly Thr Pro Gly Arg Pro Gly Ile Lys Gly
785                 790                 795                 800

Glu Pro Gly Ala Pro Gly Lys Ile Val Thr Ser Glu Gly Ser Ser Met
                805                 810                 815

Leu Thr Val Pro Gly Pro Pro Gly Pro Pro Gly Ala Met Gly Pro Pro
            820                 825                 830

Gly Pro Pro Gly Ala Pro Gly Pro Ala Gly Pro Ala Gly Leu Pro Gly
        835                 840                 845

His Gln Glu Val Leu Asn Leu Gln Gly Pro Pro Gly Pro Pro Gly Pro
    850                 855                 860

Arg Gly Pro Pro Gly Pro Ser Ile Pro Gly Pro Pro Gly Pro Arg Gly
865                 870                 875                 880

Pro Pro Gly Glu Gly Leu Pro Gly Pro Pro Gly Pro Pro Gly Ser Phe
                885                 890                 895

Leu Ser Asn Ser Glu Thr Phe Leu Phe Gly Pro Pro Gly Pro Pro Gly
            900                 905                 910

Pro Pro Gly Pro Lys Gly Asp Gln Gly Pro Pro Gly Pro Arg Gly His
        915                 920                 925

Gln Gly Glu Gln Gly Leu Pro Gly Phe Ser Thr Ser Gly Ser Ser Ser
    930                 935                 940

Phe Gly Leu Asn Leu Gln Gly Pro Pro Gly Pro Pro Gly Pro Gln Gly
945                 950                 955                 960

Pro Lys Gly Asp Lys Gly Asp Pro Gly Val Pro Gly Ala Leu Gly Ile
                965                 970                 975

Pro Ser Gly Pro Ser Glu Gly Gly Ser Ser Ser Thr Met Tyr Val Ser
            980                 985                 990

Gly Pro Pro Gly Pro Pro Gly Pro  Pro Gly Pro Pro Gly  Ser Ile Ser
        995                 1000                 1005

Ser Ser  Gly Gln Glu Ile Gln  Gln Tyr Ile Ser Glu  Tyr Met Gln
    1010                 1015                 1020

Ser Asp  Ser Ile Arg Ser Tyr  Leu Ser Gly Val Gln  Gly Pro Pro

1025                1030                1035

Gly Pro Pro Gly Pro Pro Gly Pro Val Thr Thr Ile Thr Gly Glu
    1040                1045                1050

Thr Phe Asp Tyr Ser Glu Leu Ala Ser His Val Val Ser Tyr Leu
    1055                1060                1065

Arg Thr Ser Gly Tyr Gly Val Ser Leu Phe Ser Ser Ser Ile Ser
    1070                1075                1080

Ser Glu Asp Ile Leu Ala Val Leu Gln Arg Asp Asp Val Arg Gln
    1085                1090                1095

Tyr Leu Arg Gln Tyr Leu Met Gly Pro Arg Gly Pro Pro Gly Pro
    1100                1105                1110

Pro Gly Ala Ser Gly Asp Gly Ser Leu Leu Ser Leu Asp Tyr Ala
    1115                1120                1125

Glu Leu Ser Ser Arg Ile Leu Ser Tyr Met Ser Ser Ser Gly Ile
    1130                1135                1140

Ser Ile Gly Leu Pro Gly Pro Pro Gly Pro Pro Gly Leu Pro Gly
    1145                1150                1155

Thr Ser Tyr Glu Glu Leu Leu Ser Leu Leu Arg Gly Ser Glu Phe
    1160                1165                1170

Arg Gly Ile Val Gly Pro Pro Gly Pro Pro Gly Pro Pro Gly Ile
    1175                1180                1185

Pro Gly Asn Val Trp Ser Ser Ile Ser Val Glu Asp Leu Ser Ser
    1190                1195                1200

Tyr Leu His Thr Ala Gly Leu Ser Phe Ile Pro Gly Pro Pro Gly
    1205                1210                1215

Pro Pro Gly Pro Pro Gly Pro Arg Gly Pro Pro Gly Val Ser Gly
    1220                1225                1230

Ala Leu Ala Thr Tyr Ala Ala Glu Asn Ser Asp Ser Phe Arg Ser
    1235                1240                1245

Glu Leu Ile Ser Tyr Leu Thr Ser Pro Asp Val Arg Ser Phe Ile
    1250                1255                1260

Val Gly Pro Pro Gly Pro Pro Gly Pro Gln Gly Pro Pro Gly Asp
    1265                1270                1275

Ser Arg Leu Leu Ser Thr Asp Ala Ser His Ser Arg Gly Ser Ser
    1280                1285                1290

Ser Ser Ser His Ser Ser Ser Val Arg Arg Gly Ser Ser Tyr Ser
    1295                1300                1305

Ser Ser Met Ser Thr Gly Gly Gly Gly Ala Gly Ser Leu Gly Ala
    1310                1315                1320

Gly Gly Ala Phe Gly Glu Ala Ala Gly Asp Arg Gly Pro Tyr Gly
    1325                1330                1335

Thr Asp Ile Gly Pro Gly Gly Gly Tyr Gly Ala Ala Ala Glu Gly
    1340                1345                1350

Gly Met Tyr Ala Gly Asn Gly Gly Leu Leu Gly Ala Asp Phe Ala
    1355                1360                1365

Gly Asp Leu Asp Tyr Asn Glu Leu Ala Val Arg Val Ser Glu Ser
    1370                1375                1380

Met Gln Arg Gln Gly Leu Leu Gln Gly Met Ala Tyr Thr Val Gln
    1385                1390                1395

Gly Pro Pro Gly Gln Pro Gly Pro Gln Gly Pro Pro Gly Ile Ser
    1400                1405                1410

Lys Val Phe Ser Ala Tyr Ser Asn Val Thr Ala Asp Leu Met Asp
    1415                1420                1425

Phe Phe Gln Thr Tyr Gly Ala Ile Gln Gly Pro Pro Gly Gln Lys
    1430                1435                1440

Gly Glu Met Gly Thr Pro Gly Pro Lys Gly Asp Arg Gly Pro Ala
    1445                1450                1455

Gly Pro Pro Gly His Pro Gly Pro Pro Gly Pro Arg Gly His Lys
    1460                1465                1470

Gly Glu Lys Gly Asp Lys Gly Asp Gln Val Tyr Ala Gly Arg Arg
    1475                1480                1485

Arg Arg Arg Ser Ile Ala Val Lys Pro
    1490                1495

<210> 2
<211> 13
<212> PRT
<213> artificial

<220>
<223> Epitop der Antikörper NC16a-1 und -2

<400> 2

Gly Asp Ser Met Asp Arg Ile Glu Lys Asp Arg Leu Gln

1                   5                        10

<210>  3
<211>  13
<212>  PRT
<213>  artificial

<220>
<223>  Epitop des Antikörpers NC16a-3

<400>  3

Gln Glu Glu Leu Trp Met Phe Val Arg Lys Lys Leu Met
1               5                   10

## Beschreibung

Die Erfindung betrifft das Gebiet der Diagnostik und Therapie von Hauterkrankungen, insbesondere von blasenbildenden Hauterkrankungen wie zum Beispiel bullösen Autoimmundermatosen.

### Wissenschaftlicher Hintergrund:

Kollagen XVII (auch BP180 oder BPAG-2 genannt) ist ein transmembranöses 180kDa-Glykoprotein, das in den Basalzellen diverser Plattenepithelien gebildet wird und für die Haftung und Wechselwirkung dieser Zellen mit ihrer Umgebung wichtig ist. Es kommt insbesondere in den basalen Keratinozyten der Haut und der hautnahen Schleimhäute (einschließlich Augenschleimhaut, Respirations-, Gastrointestinal- und Urogenitaltrakt) vor, aber auch in Plazenta, Nabelschnur, Kornea, Herzmuskelzellen und in einigen Zellen des Zentralnervensystems.

Die funktionale Bedeutung des Kollagen XVII für den Zusammenhalt von Epithelien mit der Basalmembran wird indirekt durch seine Rolle bei blasenbildenden Hautkrankheiten deutlich. Beim bullösen Pemphigoid, der häufigsten bullösen Autoimmundermatose, führen Autoantikörper gegen Kollagen XVII zur dessen Zerstörung und es kommt zur Trennung der Epidermis und der Dermis, und zur Blasenbildung als klinisches Symptom. Bei der erblichen junktionalen Epidermolysis bullosa bewirken Mutationen im Kollagen XVII-Gen die Ausbildung eines funktionell minderwertigen Kollagen XVII. Als Folge davon ist die Resistenz der Haut gegen äußere Scherkräfte reduziert und es kommt nach minimalster Traumatisierung zu einer Trennung von Epidermis und Dermis und somit rezidivierend zu Blasenbildung, Hautfragilität und Wundheilungsstörungen.

Die Extrazellulärdomäne von Kollagen XVII wird durch Enzyme der ADAMs-Familie physiologisch abgespalten und liegt dann als eine lösliche 120kDa-Kurzform vor. Vermutlich spielt diese lösliche Form eine Rolle bei der Zellmigration, -differenzierung und -invasion, zum Beispiel bei der Wundheilung und bei der Invasion von Krebszellen.

Eine Aufgabe der vorliegenden Erfindung ist es, Mittel bereit zu stellen, mit denen erbliche blasenbildende Dermatosen, beispielsweise junktionale Formen der Epidermolysis bullosa, diagnostiziert und bullöse Autoimmundermatosen, beispielsweise das bullöse Pemphigoid, untersucht werden können.

Es wurde gefunden, dass bestimmte Antikörper gegen humanes Kollagen XVII hervorragend als Marker für die Basalzellschicht und die Basalmembran geeignet sind. Die vorliegende Erfindung betrifft daher monoklonale Antikörper gegen menschliches Kollagen XVII, die dadurch gekennzeichnet sind, dass sie die Transmembranform von Kollagen XVII erkennen.

Parikka et al.³ beschreiben polyklonale Antikörper gegen Kollagen XVII. Im Stand der Technik der Technik wurden auch monoklonale Antikörper gegen menschliches bzw. bovines Kollagen XVII beschrieben (Hirako et al.², Marinkovich et al.⁵, Zone et al.⁶). Diese Antikörper haben den Nachteil, dass sie die Transmembranform von Kollagen XVII nicht erkennen, sondern nur die Kurzformen. Darüber hinaus sind die Hybridome dieser Antikörper nicht öffentlich zugänglich.

Der Ausdruck "Antikörper" im Sinne der vorliegenden Anmeldung bezeichnet ein Immunglobulin oder ein Derivat davon mit der gleichen Bindungsspezifität. Die Antikörper der vorliegenden Erfindung sind monoklonale Antikörper. Der Ausdruck "Antikörper", wie er hierin verwendet wird, umfasst weiterhin Derivate wie z. B. Fab-, F(ab')₂-, Fv- oder scFv-Fragmente (siehe beispielsweise Harlow and Lane, "Antibodies, a Laboratory Manual" CSH Press 1988, Cold Spring Harbor N.Y.). Der erfindungsgemäße Antikörper kann chemisch modifiziert sein. Der Antikörper kann eine Markierung enthalten, die den Nachweis des Antikörpers erleichtert. Der erfindungsgemäße Antikörper umfasst sowohl in Mäusen hergestellte Antikörper als auch davon abgeleitete teilweise oder vollständig humanisierte Varianten.

In einer bevorzugten Ausführungsform bindet der monoklonale Antiköper an die NC16a-Domäne von humanem Kollagen XVII. Die NC16a-Domäne besteht aus den Aminosäuren 490-567 der in SEQ ID NO:1 dargestellten Aminosäuresequenz. Bevorzugter bindet der Antikörper an die Aminosäuren 490 bis 523 von SEQ ID NO:1. Diese Aminosäuren stellen den N-terminalen Bereich der NC16a-Domäne von humanem Kollagen XVII dar. Noch bevorzugter sind monoklonale Antikörper, die an die in SEQ ID NO:2 gezeigte Sequenz binden. Beispiele für derartige Antiköper sind die in der vorliegenden Patentanmeldung beschriebenen Antikörper NC16a-1 und NC16a-2.

In einer anderen Ausführungsform bindet der monoklonale Antiköper an die Aminosäuren 525 bis 567 von SEQ ID NO:1, also gegen den C-terminalen Teil der NC16a-Domäne von humanem Kollagen XVII. Bevorzugter bindet ein solcher Antiköper an die in SEQ ID NO:3 dargestellte Aminosäuresequenz. Ein Beispiel für einen derartigen Antiköper ist der in der vorliegenden Anmeldung beschriebene Antiköper NC16a-3.

Der erfindungsgemäße Antikörper erkennt vorzugsweise natives Kollagen XVII in lmmunfluoreszenz und lmmunhistochemie auf kryofixierter Haut. In einer besonderen Ausführungsform erkennt der Antikörper auch Kollagen in Paraffinschnitten. In einer weiteren Ausführungsform ist der erfindungsgemäße Antikörper in der Lage, die Spaltung der Shedding-Schnittstelle (z.B. durch ein der mehrere Enzyme der ADAMs-Familie) in vitro und/oder in vivo zu inhibieren.

Die monoklonalen Antikörper der vorliegenden Erfindung können verschiedene Isotypen aufweisen, beispielsweise IgG, IgM, IgA, IgE. Vorzugsweise ist der Antikörper vom Isotyp IgG, beispielsweise vom Subtyp IgG1 oder IgG2a.

Vorzugsweise ist der monoklonale Antikörper der vorliegenden Erfindung ein isolierter monoklonaler Antikörper, d.h. er liegt im wesentlichen rein vor. Der Ausdruck "im wesentlichen rein" bedeutet, dass der Antikörper wenigstens 75 %, vorzugsweise wenigstens 90 %, bevorzugter wenigstens 95 %, am bevorzugtesten wenigstens 99 % rein ist. Die Reinheit des Antikörpers kann bestimmt werden durch an sich bekannte Techniken. Verfahren zur Reinigung eines monoklonalen Antikörpers, beispielsweise mittels Protein-G-Sepharose, sind ebenfalls bekannt.

Vorzugsweise bindet der Antikörper spezifisch an die oben genannten Sequenzen, Peptide, Proteine oder Epitope. Die Bindungsaffinität (Ka) des Antikörpers zu dem Peptid, Protein oder Epitop ist üblicherweise wenigstens 10⁶ M⁻¹ oder wenigstens 10⁷ M⁻¹, oder wenigstens 10⁸ M⁻¹ oder wenigstens 10⁹ M.

Die Erfindung betrifft weiterhin eine Hybridomzelllinie, die einen hierin beschriebenen monoklonalen Antikörper produziert. Die bevorzugtesten Hybridomzelllinien der Erfindung sind die, die die Antiköper NC16a-1, -2 bzw. -3 produzieren. Vorzugsweise ist die Hybridomzelllinie eine stabile Hybridomzelllinie. Stabile Hybridomzelllinien können durch mehrfache Klonierung hergestellt werden. Die die Antikörper NC16a-1 bzw. NC16a-3 produzierenden Hybridomzelllinien wurden bei einer anerkannten internationalen Hinterlegungsstelle gemäß den Bestimmungen des Budapester Vertrags hinterlegt (siehe Beispiel 1).

Die Erfindung betrifft auch ein Verfahren zur Herstellung eines erfindungsgemäßen Antikörpers, welches umfasst, dass man die oben beschriebenen Hybridomzellen unter Bedingungen kultiviert, die die Sekretion des Antiköpers in das Wachstumsmedium erlauben, und den Antikörper aus dem Medium gewinnt. Verfahren zur Kultivierung der Zellen und zur Gewinnung von Antikörpern aus dem Medium sind an sich bekannt (Harlow and Lane, Antibodies, a Laboratory Manual, CSH Press 1988, Cold Spring Harbor New York).

Die Erfindung betrifft weiterhin ein Verfahren zur Herstellung eines monoklonalen Antiköpers gegen Kollagen XVII, das dadurch gekennzeichnet ist, dass man die NC16a-Domäne von humanem Kollagen XVII als Antigen zur Immunisierung einsetzt. Die NC16a-Domäne besteht aus den Aminosäuren 490 bis 567 der in SEQ ID NO:1 dargestellten Aminosäuresequenz von humanem Kollagen. Zur Immunisierung kann die vollständige NC16a-Domäne oder Fragmente davon mit wenigstens 8, vorzugsweise wenigstens 15, bevorzugter wenigstens 20, bevorzugtestens wenigstens 30 Aminosäuren eingesetzt werden.

Die vorliegende Erfindung betrifft weiterhin einen Testkit, enthaltend wenigstens einen monoklonalen Antikörper gemäß der vorliegenden Erfindung. Der Antikörper kann an eine feste Phase (z.B. eine Polystyrolplatte) oder an magnetische Kügelchen gebunden sein. Der Testkit kann gegebenenfalls einen sekundären Antiköper enthalten, der gegen den monoklonalen Antikörper gerichtet ist und somit die Detektion ermöglicht. Weiterhin kann der Testkit Reagenzien enthalten, die zum Nachweis eines Signals geeignet sind, die vom monoklonalen Antikörper gegen Kollagen XVII stammen. Beispiele für derartige Reagenzien sind üblicherweise eingesetzte Enzym-substratkombinationen wie z.B.:
a) Alkalische Phosphatase als Enzym zusammen mit den Substraten 5-Brom-4-Chlor-3-Indolylphosphat (BCIP) und Nitroblautetrazoleum (NBT)
b) Meerrettichperoxidase als Enzym und 3,3-Diaminobenzidin Tetrahydrochlorid (DAB) als Substrat.

Andere Reagenzien, die in dem Testkit enthalten sein können, können durch Immunfluoreszenz detektiert werden. Beispiele für solche Reagenzien sind Fluoresceinisothiozyanat, Phycoerythrin, Texas Red, TRIC, Cy3, oder Cy5. Andere Färbetechniken können beispielsweise Gold oder Rodamin einsetzen.

Der erfindungsgemäße Testkit kann weiterhin einen Antikörper enthalten, der als Marker für die Dermis geeignet ist, beispielsweise einen Anti-Laminin5-Antikörper.

Die bevorzugten Ausführungsformen des Testkits, insbesondere in Bezug auf den darin enthaltenen erfindungsgemäßen monoklonalen Antikörper, entsprechen den bevorzugten Ausführungsformen des monoklonalen Antikörpers, wie oben beschrieben.

Die monoklonalen Antikörper der Erfindung sind geeignet zum Nachweis von Kollagen XVII oder Derivaten davon. Die Erfindung betrifft daher auch die Verwendung eines erfindungsgemäßen monoklonalen Antikörpers zum Nachweis von Kollagen XVII oder Derivaten davon, insbesondere von humanem Kollagen XVII.

Der Begriff "Derivat von Kollagen XVII" im Sinne der vorliegenden Anmeldung umfasst chemisch oder gentechnisch modifiziertes Kollagen XVII. Der Begriff umfasst weiterhin Fragmente von Kollagen XVII, die wenigstens ein Epitop von Kollagen XVII enthalten. Die Fragmente bestehen aus wenigstens 8, vorzugsweise wenigstens 15, bevorzugter wenigstens 25 oder wenigstens 50 oder wenigstens 100 aufeinanderfolgenden Aminosäuren von Kollagen XVII. Ein bevorzugtes Fragment ist die lösliche 120 KDa-Kurzform von Kollagen XVII, die im wesentlichen aus den Aminosäuren 524-1497 besteht. Derivate von Kollagen XVII schließen auch Moleküle ein, die derartige Fragmente enthalten. Schließlich sind Derivate von Kollagen XVII auch Moleküle, die wenigstens ein Epitop von Kollagen XVII umfassen. Das Derivat von Kollagen XVII kann löslich oder unlöslich (z.B. membrangebunden) sein. Derivate von Kollagen XVII schließen auch alle allelischen Varianten von Kollagen XVII und alle Spezies-Varianten von Kollagen XVII ein.

Ein weiterer Aspekt der Erfindung ist ein Verfahren zum Nachweis von Kollagen XVII oder einem Derivat davon, das umfasst, dass man wenigstens einen erfindungsgemäßen Antikörper mit einer Probe in Kontakt bringt und anschließend bestimmt, ob sich Komplexe zwischen Antikörper und Kollagen XVII oder einem Derivat davon gebildet haben. Die Probe wird mit dem Antikörper unter Bedingungen in Kontakt gebracht, die die Bildung eines Komplexes zwischen Antikörper und Kollagen XVII oder einem Derivat davon erlauben. Die Probe kann Kollagen XVII oder ein Derivat davon enthalten. Die Bestimmung, ob sich Komplexe gebildet haben, kann qualitativ oder quantitativ erfolgen. Diese Bestimmung kann nach an sich bekannten Methoden erfolgen. Das Verfahren kann als immunhistochemischer Assay, als Immunpräzipitation, als ELISA, als Western Blot-Assay oder als anderer Assay ausgebildet sein. Verschiedene Arten von Immunassays sind in "Lottspeich und Zorbas, Bioanalytik, Spektrum Akademischer Verlag, 1998" beschrieben.

In einer weiteren Ausführungsform betrifft die Erfindung die Verwendung eines erfindungsgemäßen monoklonalen Antikörpers als Marker für die Basalzellschicht und/oder die Basalmembran. Ein weiterer Aspekt der Erfindung ist die Verwendung eines erfindungsgemäßen monoklonalen Antikörpers zur Bestimmung von **Basalmembrandefekten, vorzugsweise bei bullösen Dermatosen. Ebenso kann der** Antikörper verwendet werden zur Bestimmung der Blasenbildungsebene bei bullösen Dermatosen. Bei junktionaler Epidermolysis bullosa zeigt sich eine reduzierte oder komplett negative Anfärbung im Vergleich zur Normalhaut.

Ein weiterer Aspekt der Erfindung ist die Verwendung eines erfindungsgemäßen monoklonalen Antikörpers zum Nachweis und/oder zur Untersuchung von blasenbildenden Hauterkrankungen. Blasenbildende Hauterkrankungen schließen Autoimmundermatosen ein. Beispiele für blasenbildende Hauterkrankungen sind bullöses Pemphigoid (z. B. juveniles bullöses Pemphigoid, lokalisiertes bullöses Pemphigoid, noduläres bullöses Pemphigoid, erythrodermatisches bullöses Pemphigoid, dyshidrosiformes bullöses Pemphigoid, herpetiformes oder vesikulöses bullöses Pemphigoid) junktionale Epidermolysis bullosa, Pemphigoid gestationis, vernarbendes Schleimhautpemphigoid, Lineare lgA-Dermatose, Lichen planus pemphigoides, Pemphigus vulgaris, Pemphigus foliaceus, Epidermolysis bullosa acquisita und Dermatitis herpetiformis Duhring. Beispiele für blasenbildende hereditäre Erkrankungen sind junktionale Epidermolysis bullosa, Epidermolysis bullosa simplex und Epidermolysis bullosa dystrophicans.

Die erfindungsgemäßen Antikörper können auch bei der Diagnostik maligner Tumoren eingesetzt werden. Bei allen Tumoren in Epithelien, die Kollagen XVII exprimieren, kann die Invasion des Tumors in das Bindegewebe durch Anfärbung der Basalmembran mit Hilfe eines erfindungsgemäßen Antikörpers sichtbar gemacht werden. Mit Hilfe des Antikörpers NC16a-3 kann die Invasion des Tumors sogar auf Paraffinschnitten sichtbar gemacht werden. Bei Plattenepithelkarzinomen wurde eine Überexpression von Kollagen XVII im Rahmen der Tumorprogression beschrieben. Durch Verwendung der monoklonalen Antikörper der vorliegenden Erfindung ist hier eine genaue Beurteilbarkeit der Kollagen XVII-Expression gegeben. Die Bestimmung der Kollagen XVII-Expression kann sich auch als prognostischer Faktor und somit zur Abschätzung der individuell besten Therapiemodalität eignen.

Die Erfindung betrifft auch die Verwendung eines erfindungsgemäßen Antikörpers zur Herstellung eines Medikaments zur Vorbeugung oder Behandlung einer Hauterkrankung, vorzugsweise einer blasenbildenden Hauterkrankung. Die Hauterkrankung kann eine der oben genannten Hauterkrankungen sein. In einer besonderen Ausführungsform können die monoklonalen Antikörper durch Bindung an die NC16a-Domäne von Kollagen XVII die Shedding-Schnittstelle blockieren und so in vivo die Abspaltung der Extrazellulärdomäne verhindern. Die Erfinder konnten zeigen, dass in vitro eine Inhibition des Sheddings durch den Antikörper NC16a-1 möglich ist. Da man annimmt, dass das Shedding in der Pathogenese bullöser Autoimmunerkrankungen eine Rolle spielt, kann der Einsatz der erfindungsgemäßen Antikörper bei Patienten (z.B. mit bullösem Pemphigoid) therapeutisch wirksam sein.

Besonderes erwähnenswert ist der extrem hochaffine NC16a-3-Antikörper, da er bei entsprechender Verdünnung selektiv die Kurzform von Kollagen XVII erkennt, und daher als Marker für das Shedding der Ektodomäne eingesetzt werden kann. Desweiteren kann dieser Antikörper, nach Aufreinigung mittels Protein G-Affinitätschromatographie, in der lmmunhistochemie auf Formalin-fixiertem und Paraffin-eingebettetem Gewebe eingesetzt werden.

Die drei Antikörper können relativ einfach in großen Mengen hergestellt werden. Die antikörperhaltigen Kulturüberstände sind langfristig bei 4°C lagerbar. Die Aufreinigung über z.B. Protein G ermöglicht eine Ankonzentration, höhere Reinheit und die Möglichkeit der direkten Kopplung des Antikörpers an Enzyme, Fluorochrome oder andere Liganden.

In einer speziellen Ausführungsform betrifft die Erfindung auch Antikörper, die die Transmembranform von humanem Kollagen XVII nicht erkennen.

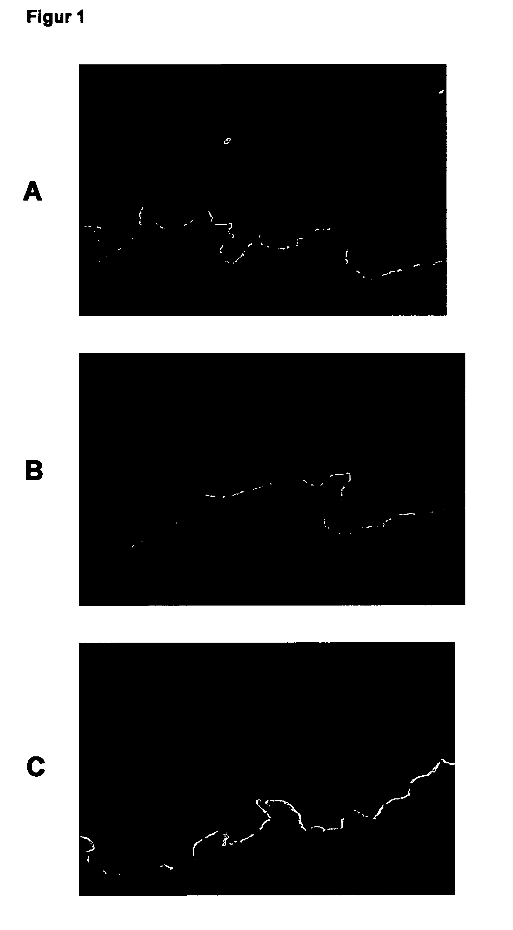

**Figur 1** zeigt Immunfluoreszenzen auf humaner NaCl-Spalthaut (siehe Beispiel 2). Die dermale Seite wurde mit einem anti-Laminin 5-Antikörper angefärbt (jeweils untere Färbung). Die epidermale Seite wurde durch die erfindungsgemäßen Antikörper NC16a-1 (A), NC16a-2 (B) und NC16a-3 (C) markiert (jeweils obere Färbung).

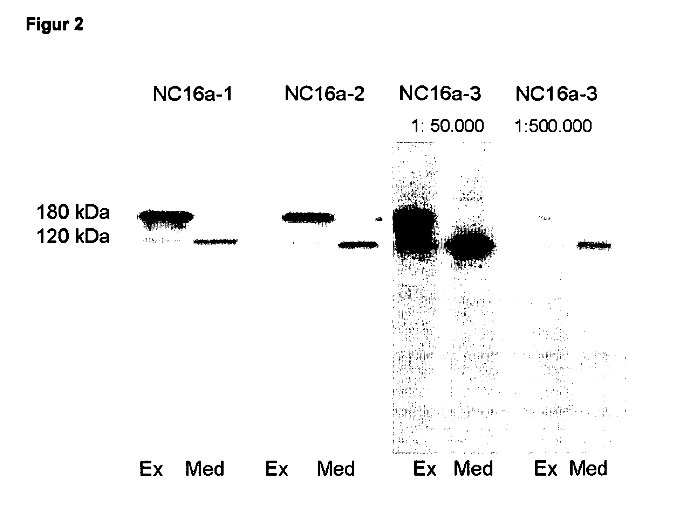

**Figur 2** zeigt die Ergebnisse eines Western-Blots mit Keratinozytenextrakt und -medium (siehe Beispiel 3).

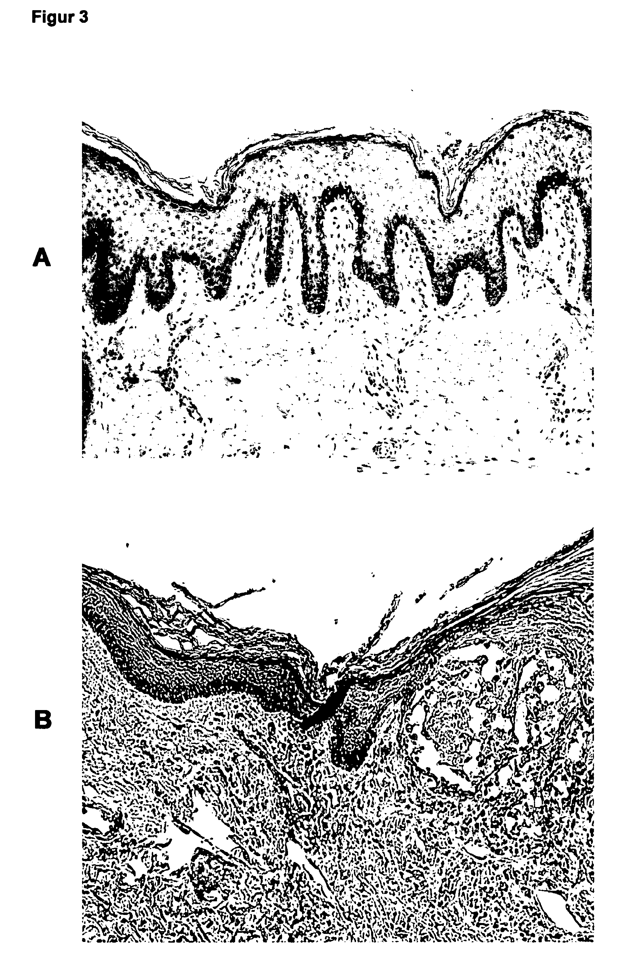

**Figur 3** zeigt lmmunhistochemie mit NC16a-3-Antikörper (siehe Beispiel 4). A: Deutliche und spezifische Anfärbung der Basalzellschicht auf Paraffinschnitten formalinfixierter normaler humaner Haut. B: Anfärbung der Basalzellschicht auf formalinfixierten Paraffinschnitten eines malignen Melanoms: im rechten Abschnitt der Abbildung zeigt sich ein Einbruch des Tumors in die Dermis.

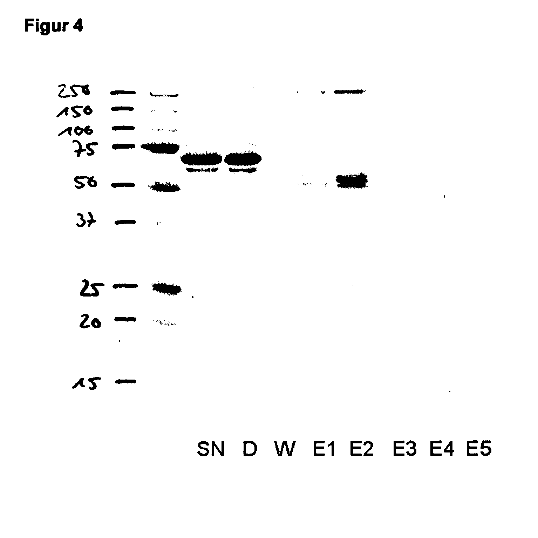

**Figur 4** zeigt die affinitätschromatographische Aufreinigung des NCl6a-1-Antikörpers mit Protein G-Sepharose 4 Fast Flow (siehe Beispiel 5). Nach SDS-Page-Elektrophorese wurde das 7% Gel mit Coomassie-Lösung gefärbt. Auf das Gel aufgetragen wurden Hybridom-Überstand (SN), Durchfluß (D), Waschpuffer (W) und die Eluatfraktionen (E1-E5).

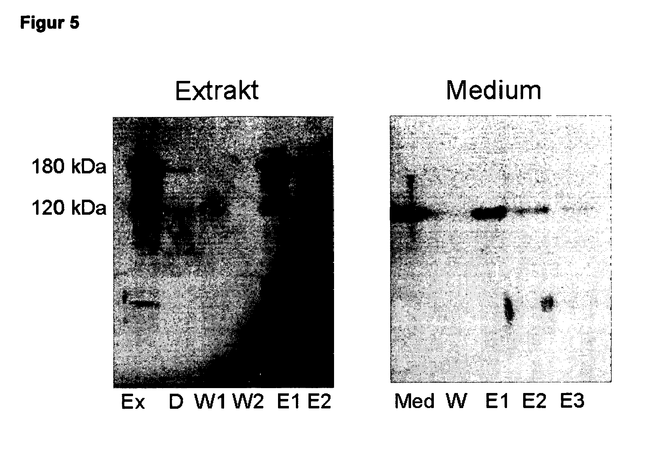

**Figur 5** zeigt das Ergebnis einer lmmunpräzipitation mit NC16a-3-Antikörper und Keratinozytenextrakt bzw. -medium (siehe Beispiel 7). Zur Kontrolle der lmmunpräzipitation wurden Ausgangsmaterial (Extrakt, Ex oder Medium, Med), Durchfluß (D), Waschpuffer (W) sowie die Eluatfraktionen (E1-E3) auf ein 7% SDS-Page-Gel aufgetragen und durch einen polyklonalen Kaninchen-Anti-NC16a-Antikörper dargestellt.

Die nachfolgenden Beispiele erläutern die Erfindung näher.

### Beispiele

### Beispiel 1. Herstellung monoklonaler Antikörper gegen Kollagen XVII

Durch Immunisierung von Balb/c-Mäusen mit rekombinanten Kollagen XVII-Fragmenten, anschließender Fusion der Maus-Lymphozyten mit Myelomzellen und Screening der entstehenden Hybridoma-Klone wurden drei verschiedene monoklonale Antikörper gegen die NC16a-Domäne (Aminosäurenreste 490-566) des Kollagen XVII hergestellt. Die NC16a- Domäne ist der membrannahe Bereich der Extrazellulärdomäne, in der sowohl die Spaltstelle der ADAMs-Proteinasen als auch die immundominanten Autoantikörper-Epitope beim Pemphigoid liegen. Es wurden prokaryotisch in E.coli hergestellte GST-Fusionsproteine zur Immunisierung eingesetzt. Die die Antikörper NC16a-1, NC16a-2 und NC16a-3 produzierenden Hybridome entstanden nach Immunisierung mit einem GST-Fusionsprotein, welches die Aminosäuren 490-567 umfasste. Weitere Mäuse wurden mit kürzeren Fragmenten (Aminosäuren 490-523 und 525-567) immunisiert.

Die rekombinanten Proteine wurden mit Freund's komplettem Adjuvans zusammen intraperitoneal gespritzt. Für die Boosts nach 4, 8 und 12 Wochen wurde Freund's inkomplettes Adjuvans zusammen mit dem jeweiligen Fusionsprotein verwendet. Drei Tage nach der letzten Immunisierung wurden die Mäuse getötet, die Milz entfernt und die Milzlymphozyten im Verhältnis 3:1 mit Myelomzellen (TW Sp2/0) unter Verwendung von Polyethylenglykol fusioniert. Die Hybridome wurden in HAT-Medium bei 37°C, 5% CO₂ kultiviert (s. Harlow and Lane, 1988). Kulturüberstände wurden mittels indirekter lmmunfluoreszenz auf normaler humaner Haut und ELlSA mit rekombinantem Kollagen XVII auf Antikörperproduktion gestestet.

Die Hybridomzelllinien, die die Antikörper NC16a-1 bzw. NC16a-3 produzieren, wurden am 25. Januar 2006 bei der Deutschen Sammlung von Mikroorganismen und Zellkulturen GmbH (DSMZ), Mascheroder Weg 1b, D-38124 Braunschweig, Deutschland, hinterlegt. Die den Antikörper NC16a-1 produzierende Hybridomzelllinie hat die Eingangsnummer DSM ACC2758 erhalten. Die den Antikörper NC16a-3 produzierende Hybridomzelllinie hat die Eingangsnummer DSM ACC2759 erhalten.

### Beispiel 2. Immunfluoreszenz

Die Antikörper NC16a-1, NC16a-2 und NC16a-3 wurden jeweils unverdünnt mit Schnitten kryofixierter humaner Haut 1 h lang inkubiert. Nach 3 Waschschritten mit 0,9% NaCl erfolgte die Inkubation mit einem Rhodamin-Ziege-anti-Maus-lgG Antikörper (Jackson, 1:80 in 0,9% NaCl, 1 h). Der Objektträger wurde erneut 3 x gewaschen und schließlich mit Bleichschutz (Mowiol) eingedeckt.

Alle 3 Antikörper zeigen auf normaler humaner Haut eine lineare Färbung entlang der Basalmembran. Auf mit 1 M NaCl-gespaltener humaner Haut reagieren die Antikörper mit der epidermalen Seite der Spaltbildung, während ein als Kontrolle verwendeter Kaninchenanti-Laminin 5 Antikörper (Immundiagnostik AG, 1:100; 2. Antikörper: FITC-Ziege-anti-Kaninchen IgG, Jackson 1:100) die dermale Seite der Blase markiert **(s. Fig.1).**

Die Intensität der Fluoreszenz variiert zwischen den monoklonalen Antikörpern, wobei der Antikörper NC16a-1 die stärkste Reaktivität mit der Basalmembran und der NC16a-3 Antikörper die schwächste Reaktivität aufweist.

Im Rahmen des Antigen Mapping zur Diagnostik hereditärer bullöser Dermatosen werden die Antikörper eingesetzt, um nachzuweisen, ob der Patient Kollagen XVII in der Haut exprimiert oder nicht. Auf Kollagen XVII-negativer Haut von Patienten, die Null-Mutationen im Kollagen XVII-Gen aufweisen, zeigt sich bei positiver Laminin-5 Färbung keine Bindung der monoklonalen Antikörper an die Basalmembran, was mit einem genetischen Ansatz die Spezifität der Antikörper bestätigt.

### Beispiel 3. Western Blot

Extrakte normaler humaner Keratinozyten und konditioniertes Keratinozytenmedium (10 µl je Geltasche) wurden in einem 4,5-15% Gradienten-SDS-Page-Gel elektrophoretisch aufgetrennt und anschließend auf Nitrocellulose transferiert (Methodik siehe *Schäcke et al¹*). Nach Blockierung mit 2% Milchpulver in TBS-Puffer pH 7,5 über 30 min wurden die Nitrocellulosestreifen jeweils mit NC16a-1 (bis 1:80 in Blockierungspuffer), NC16a-2 (unverdünnt) und NC16a-3 (bis 1:500.000) über Nacht bei Raumtemperatur inkubiert. Nach 5 Waschschritten mit TBS wurden die Nitrozellulosestreifen mit dem Sekundärantikörper (HRP-Ziege-anti-Maus-igG (BioRad, 1:3000 in TBS) inkubiert. Die Entwicklung erfolgte mittels Chemilumineszenz (Detektionsreagenz ECL plus, Amersham).

Bei allen 3 monoklonalen Antikörpern konnte Reaktivität mit der Transmembranform (180kDa) von Kollagen XVII und der Kurzform (120kDa-Ektodomäne) nachgewiesen werden **(s. Fig. 2)**. Es konnte gezeigt werden, dass der hochaffine NC16a-3-Antikörper bei entsprechend hoher Verdünnung (1:500.000 in TBS) selektiv die Kurzform von Kollagen XVII erkennt und somit als Marker für das Shedding der Ektodomäne eingesetzt werden kann.

### Beispiel 4. Immunhistochemische Färbungen

Für die Immunhistochemie wurden formalinfixierte Paraffinschnitte humaner Haut nach Vorbehandlung mit Target Retrieval pH 9 (DAKO) zur Antigendemaskierung verwendet. Der NC16a-3-Antikörper wurde 1:2 in 1% BSA in PBS verdünnt und über Nacht bei 4°C mit den Schnitten inkubiert. Als Sekundärantikörper wurde das DAKO Cytomation EnVision+ System-HRP labelled polymer Anti-Mouse und zur Entwicklung AEC+ High Sensitivity Substrate Chromogen (DAKO) entsprechend den Empfehlungen des Herstellers verwendet.

Während immunhistochemische Färbungen auf kryofixierter Haut mit allen 3 monoklonalen Antikörpern jeweils eine deutliche, lineare Anfärbung der Basalmembran ergeben, eignet sich zur Immunhistochemie auf formalinfixierter Haut nur der NC16a-3-Antikörper. Hierbei zeigt sich eine perizelluläre Anfärbung der Basalzellschicht. Diese Markierung der Basalzellschicht kann man sich auf Gewebeschnitten epidermaler Tumoren zunutze machen, um den Durchbruch des Tumors in die Dermis und damit beginnende Invasivität des Tumors sichtbar zu machen **(s. Fig. 3).**

### Beispiel 5. Aufreinigung der monoklonalen Antikörper mittels Protein-G-Sepharose

50ml Hybridom-Überstand (Supernatant, SN) wurde über eine Säule mit 1ml Protein G-Sepharose 4 Fast Flow (Amersham) gegeben. Die nichtgebundene Fraktion (Durchfluß) wurde asserviert. Es folgte ein Waschschritt mit 5 x 10ml Waschpuffer (Protokoll und Puffer entsprechend den Anleitungen von Amersham). Die Elution erfolgte in 5 Schritten mit jeweils 1000 µl 0,1M Glycin, pH 2,7, wobei das Eluat sofort in 200 µl Neutralisations-Puffer (1M Tris-HCI, pH 9,0) aufgefangen wurde. Zur Kontrolle der Aufreinigung wurden Ausgangsmaterial (SN), Durchfluß (D), Waschpuffer (W) und die Eluatfraktionen 1-5 (E1-E5) in einem Coomassie-Gel unter reduzierten und nicht reduzierten Bedingungen dargestellt **(s. Fig. 4)**.

In den Eluatfraktionen E1-E3 lassen sich die Antikörper als eine ca. 50kDa (schwere Immunglobulin-Ketten) sowie eine 28 kDa-Bande (leichte Immunglobulin-Ketten) unter reduzierten Bedingungen nachweisen. Die antikörperhaltigen Eluatfraktionen E1-E3 wurden vereinigt und die Konzentration dieses Eluates mittels eines modifizierten Protein-Assay nach Lowry bestimmt. Hierbei ergaben sich für alle 3 monoklonalen Antikörper ähnliche Werte um 0,16 mg/ml. Aus 50ml Hybridom-Überstand konnten insgesamt ca. 0,5mg Antikörper aufgereinigt werden.

### Beispiel 6. ELISA

Für ELISA-Untersuchungen wurden verschiedene rekombinante bakterielle GST-NC16a-Fusionsproteine (Proteinexpression s. *Schumann et al⁴*), welche entweder die gesamte NC16a-Domäne (Aminosäuren 490-567 = NC16a), die aminoterminale Hälfte der NC16a-Domäne (Aminosäuren 490-523 = NC16a-N) oder die carboxyterminale Hälfte der NC16a-Domäne (Aminosäuren 525-567 = NC16a-C) umfassten, verwendet. Mikrotiterplatten wurden mit 0,5µg eines NC16a-Proteins in 100µl NaHCO₃ je well mind. 5 h beschichtet, anschließend gewaschen, mit 2% Milchpulver in TBS + 0,05% Tween (TBST) für 2 h geblockt und mit den monoklonalen Antikörpern (unverdünnt) über Nacht inkubiert. Nach 5 Waschschritten mit TBST erfolgte die Inkubation mit HRP-Ziege-anti-Maus-Antikörper (BioRad; 1:6000 in TBS) und Entwicklung mit ABTS (2,2Azino-bis (3-Ethylbenzthiazolon-6-Sulfonic Acid; Sigma). Die Optische Dichte wurde bei 405nm mittels eines Microplate-Reader gemessen. Werte < 0,1 OD entsprechen dem Leerwert (negativ).

Die gemessenen optischen Dichten sind in der folgenden Tabelle dargestellt:

| | NC16a (AA 490-567) | NC16a-N (AA 490-523) | NC16a-C AA 525-567 |
|---|---|---|---|
| NC16a-1 | **0,78** | **0,69** | 0,07 |
| NC16a-2 | **0,82** | **0,67** | 0,07 |
| NC16a-3 | **0,75** | 0,06 | **0,76** |

Während alle drei Antikörper mit dem Gesamt-NC16a-Protein reagieren, weisen die Antikörper NC16a-1 und NC16a-2 Spezifität für den aminoterminalen Abschnitt der NC16a-Domäne (entsprechend dem NC16a-N-Protein) und der Antikörper NC16a-3 Spezifität für den carboxyterminalen Abschnitt der NC16a-Domäne (entsprechend dem NC16a-C-Protein) auf.

### Beispiel 7. Immunpräzipitation

Nach Vorreinigung von 200µl Keratinozytenextrakt (Ex) bzw. 10ml Keratinozytenmedium (Med) mit jeweils 100µl Protein-G-Sepharose wurden Extrakt bzw. Medium mit 200µl des monoklonalen NC16a-3-Antikörpers über Nacht bei 4°C auf dem Überkopfschüttler inkubiert. Den Antigen-Antikörper-Komplexen wurde dann jeweils 300µl frische Sepharose hinzugefügt (Inkubation für 1 h bei 4°C). Durch Zentrifugation mit 3500 g bei 4°C für 15 Minuten wurden die an die Sepharose gebundenen Antigen-Antikörper-Komplexe (Pellet) von den sonstigen, löslichen Bestandteilen der Extrakte und des Mediums (Durchfluß, D) separiert. Die Sepharose wurde mit 3 x 10ml Tris/100mM NaCl /NP40-Puffer pH 7,4 gewaschen. Anschließend erfolgte die Elution der Kollagen XVII-Proteine durch Inkubation mit 2-3 x 100 µl SDS-Page Probenpuffer und anschließender Zentrifugation. Ausgangsmaterial (Ex bzw. Med), Durchfluß (D), Waschpuffer (W) und Eluate (E1-E3) wurden auf einem 7% SDS-Page-Gel elektrophoretisch aufgetrennt und auf Nitrozellulose transferiert. Das immunpräzipitierte Kollagen XVII wurde mit einem polyklonalen Kaninchen-anti-NC16a-Antikörper detektiert **(s. Fig. 5)**.

Die Immunpräzipitation mit dem NC16a-3 Antikörper ermöglicht eine Aufreinigung des Kollagen XVII-Proteins aus Keratinozytenextrakten sowie der 120kDa-Kurzform aus konditioniertem Medium.

### Beispiel 8. Epitopcharakterisierung

Bisher hergestellte monoklonale Antikörper gegen Kollagen XVII (siehe *Hirako et al², Marinkovich et al⁵* und *Zone et al⁶)* sind hinsichtlich der Epitope nicht genau charakterisiert. Darüber hinaus sind diese Antikörper, auch im Rahmen wissenschaftlicher Kooperationen, kaum erhältlich.

Unsere Epitopcharakterisierung erfolgte mittels synthetischer Peptide (PepSPOT-Technologie, JPT Peptide Technologies GmbH, Berlin). Die gesamte NC16a-Domäne von Kollagen XVII wurde durch synthetische 13-mer-Peptide, welche jeweils um 2 Aminosäuren überlappten, abgebildet. Die Peptide wurden an Whatman Cellulose-Folie kovalent gebunden und dann mit den monoklonalen Antikörpern inkubiert (Protokoll analog zum Western Blot von Beispiel 3). Die Entwicklung erfolgte mittels Chemilumineszenz (ECL, Amersham). Die Peptide, mit denen der jeweilige Antikörper reagierte, konnten anhand der Sequenz-Liste der PepSPOTs klar einer bestimmten Aminosäuresequenz zugeordnet werden:
NC16a-1 513GDSMDRIEKDRLQ525 (SEQ ID NO:2)
NC16a-2 513GDSMDRIEKDRLQ525 (SEQ ID NO:2)
NC16a-3 546QEELWMFVRKKLM558 (SEQ ID NO:3)

Diese Ergebnisse korrelieren mit den Resultaten aus der ELlSA-Untersuchung mit verschiedenen NC16a-Fragmenten (siehe Beispiel 6. ELISA).

### Beispiel 9. Inhibition des Sheddings durch monoklonale Antikörper

Erste in vitro-Experimente mit HaCaT-Keratinozyten weisen interessanterweise darauf hin, dass der NC16a-1-Antikörper, nicht aber der NC16a-3-Antikörper, inhibitorisch auf den Shedding-Prozess einwirkt: nach Zugabe des NC16a-1-Antikörpers zu HaCaT-Keratinozyten war bei gleichen Mengen an 180kDa-Kollagen XVII signifikant weniger 120kDa-Ektodomäne im Medium nachweisbar als bei der Kontrollgruppe oder dem NC16a-3-Antikörper. Aufgrund der Epitop-Homologie ist zu erwarten, dass auch der NC16a-2-Antikörper das Shedding hemmen kann.

Die Inhibition des Sheddings durch die Autoantikörper kann an humanen Keratinozyten überprüft werden. Aus diesen Versuchen können sich therapeutische Konsequenzen mit dem Ziel einer spezifischen Behandlung bullöser Autoimmundermatosen ergeben. Desweiteren bietet ein Antikörper, welcher spezifisch die Abspaltung der Kollagen XVII-Extrazellulärdomäne hemmen kann, ideale Voraussetzungen, um die Rolle des Sheddings auf die physiologische Regulation der Keratinozyten zu analysieren.

### Referenzen:

1 Schäcke H, Schumann H, Hammami-Hauasli N, Raghunath M, Bruckner-Tuderman L: Two forms of collagen XVII in keratinocytes. A full-lenght transmembrane protein and a soluble ectodomain. J Biol Chem 1998; 273: 25937-25943.
2 Hirako Y, Usukura J, Uematsu J, Hashimoto T, Kitajima Y, Owaribe K: Cleavage of BP180, a 180-kDa bullous pemphigoid antigen, yields a 120-kDa collagenous extracellular polypeptide. J Biol Chem 1998; 273: 9711-9717.
3 Parikka M, Kainulainen T, Tasanen K, Väänänen A, Bruckner-Tuderman L, Salo T: Alterations of collagen XVII expression during transformation of oral epithelium to dysplasia and carcinoma. J Histochem Cytochem 2003; 51: 921-929.
4 Schumann H, Baetge J, Tasanen K, Wojnarowska F, Schäcke H, Zillikens D, Bruckner-Tuderman L: The shed ectodomain of collagen XVII / BP180 is targeted by autoantibodies in different blistering skin diseases. Am J Pathol 2000; 156:685-695.
5 Marinkovich MP, Taylor TB, Keene DR, Burgeson RE, Zone JJ: LAD-1, the linear 1gA-bullous dermatosis autoantigen, is a novel 120kDa-anchoring filament protein synthesized by epidermal cells. J Invest Dermatol 1996; 106: 734-738.
6 Zone JJ, Taylor TB, Meyer LJ, Petersen MJ: The 97-kDa linear IgA bullous disease antigen is identical to a portion of the extracellular domain of the 180-kDa bullous pemphigoid antigen, BPAg2. J Invest Dermatol 1998; 110: 207-210.

## Patentansprüche

1. Monoklonaler Antikörper gegen humanes Kollagen XVII, **dadurch gekennzeichnet, dass** er die Transmembranform von Kollagen XVII erkennt.

2. Monoklonaler Antikörper gegen humanes Kollagen XVII, **dadurch gekennzeichnet, dass** er gegen die NC16a-Domäne von humanem Kollagen XVII gerichtet ist.

3. Monoklonaler Antikörper nach Anspruch 1 oder 2, **dadurch gekennzeichnet, dass** er gegen die Aminosäuren 490-523 von SEQ ID N0:1 gerichtet ist.

4. Monoklonaler Antikörper nach einem der Ansprüche 1 bis 3, **dadurch gekennzeichnet, dass** er gegen die Sequenz SEQ ID NO:2 gerichtet ist.

5. Monoklonaler Antikörper nach Anspruch 1 oder 2, **dadurch gekennzeichnet, dass** er gegen die Aminosäuren 525-567 von SEQ ID NO:1 gerichtet ist.

6. Monoklonaler Antikörper nach Anspruch 5, **dadurch gekennzeichnet, dass** er gegen die Sequenz SEQ ID NO:3 gerichtet ist.

7. Hybridom-Zellinie, die einen monoklonalen Antikörper nach einem der Ansprüche 1 bis 6 produziert.

8. Hybridom-Zellinie nach Anspruch 7, **dadurch gekennzeichnet, dass** es eine stabile Hybridom-Zellinie ist.

9. Verfahren zur Herstellung eines monoklonalen Antikörpers gegen Kollagen XVII, **dadurch gekennzeichnet, dass** man die NC16a-Domäne von humanem Kollagen XVII als Antigen zur Immunisierung einsetzt.

10. Testkit, enthaltend wenigstens einen monoklonalen Antikörper nach einem der Ansprüche 1 bis 6.

11. Verfahren zum Nachweis von Kollagen XVII oder einem Derivat davon, **dadurch gekennzeichnet, dass** man eine Probe mit einem monoklonalen Antikörper nach einem der Ansprüche 1 bis 6 in Kontakt bringt und die Menge an Komplex aus Antikörper und Kollagen XVII oder aus Antikörper und Kollagen XVII-Derivat bestimmt.

12. Verwendung eines monoklonalen Antikörpers nach einem der Ansprüche 1 bis 6 zum Nachweis von Kollagen XVII oder einem Derivat davon.

13. Verwendung eines monoklonalen Antikörpers nach einem der Ansprüche 1 bis 6 als Marker für die Basalzellschicht und/oder die Basalmembran.

14. Verwendung eines monoklonalen Antikörpers nach einem der Ansprüche 1 bis 6 zur Bestimmung von Basalmembrandefekten.

15. Verwendung eines monoklonalen Antikörpers nach einem der Ansprüche 1 bis 6 zum Nachweis und/oder zur Untersuchung von blasenbildenden Hauterkrankungen.

16. Verwendung nach Anspruch 16, **dadurch gekennzeichnet, dass** die blasenbildende Hauterkrankung eine Autoimmundermatose ist.

17. Verwendung nach Anspruch 15 oder 16, **dadurch gekennzeichnet, dass** die blasenbildende Hauterkrankung ausgewählt ist aus der Gruppe bestehend aus bullösem Pemphigoid, junktionaler Epidermolysis bullosa, Pemphigoid gestationis, vernarbendes Schleimhautpemphigoid, Lineare IgA-Dermatose, Lichen planus pemphigoides, Pemphigus vulgaris, Pemphigus foliaceus, Epidermolysis bullosa acquisita, Dermatitis herpetiformis Duhring, Epidermolysis bullosa, Epidermolysis bullosa simplex und Epidermolysis bullosa dystrophicans.

18. Verwendung eines monoklonalen Antikörpers nach einem der Ansprüche 1 bis 6 zur Untersuchung epithelialer Tumoren.

19. Verwendung eines monoklonalen Antikörpers nach einem der Ansprüche 1 bis 6 zur Herstellung eines Medikaments zur Vorbeugung oder Behandlung einer Hauterkrankung.
